# EUROPEAN PATENT APPLICATION

(11) **EP 4 358 090 A1**
(43) Date of publication of application: **24.04.2024**
(21) Application number: 23752252.9
(22) Date of filing: 29.01.2023
(51) Int. Cl.: G16H 30/20, G06F 16/532

(54) **IMAGE DISPLAY METHOD AND APPARATUS, AND TERMINAL AND STORAGE MEDIUM**

(30) Priority: 11.02.2022 CN 202210129761
(71) Applicant: Guangzhou LBP Medicine Science & Technology Co., Ltd, Guangzhou, Guangdong 510799 (CN)
(72) Inventor: ZHANG, Hao, Guangzhou, Guangdong 510799 (CN); PENG, Zhenwu, Guangzhou, Guangdong 510799 (CN)
(74) Representative: Yang, Shu
(86) International application number: PCT/CN2023/073721
(87) International publication number: WO 2023/151472

(57) **Abstract**

An image display method and apparatus, and a terminal and a storage medium; and same belong to the technical field of computers. The method comprises: displaying an object information interface, wherein the object information interface includes at least one object (401); in response to a selection operation on a target object in the object information interface, acquiring a pathological image of the target object (402); determining, from the pathological image, at least one pathological sub-image which belongs to a target type (403); and in the object information interface, displaying the at least one pathological sub-image which belongs to the target type (404). By means of determining, from a pathological image, a pathological sub-image which belongs to a target type, and then displaying the pathological sub-image which belongs to the target type, key information with a higher reference value in the pathological image is displayed, such that the pertinence is increased, and the display effect of a pathological image is better.

## Description

This application claims priority to Chinese Patent Application No. 202210129761.1 filed on February 11, 2022 and entitled "IMAGE DISPLAY METHOD AND DEVICE, TERMINAL AND STORAGE MEDIUM," the disclosure of which is herein incorporated by reference in its entirety.

### TECHNICAL FIELD

The present disclosure relates to the field of computer technologies, and in particular, relates to a method and apparatus for displaying images, a terminal, and a storage medium.

### BACKGROUND

With the rapid development of the image technologies, a pathological image of an object is acquired by shooting or scanning the object. The pathological image is an image for displaying pathological morphologies of body organs, tissues or cells of the object.

In some practices, upon acquisition of the pathological image of the object, the pathological image is directly displayed to the user, and the display effect is poor.

### SUMMARY

Embodiments of the present disclosure provide a method and apparatus for displaying images, an electronic device, and a storage medium, which can improve the display effect of the pathological image. The technical solutions are as follows.

In one aspect, a method for displaying images is provided. The method includes:
displaying an object information interface, wherein the object information interface includes at least one object;
acquiring a pathological image of a target object in response to a select operation on the target object on the object information interface;
determining at least one pathological sub-image of a target type from the pathological image; and
displaying the at least one pathological sub-image of the target type on the object information interface.

In another aspect, an apparatus for displaying images is provided. The apparatus includes:
an interface displaying module, configured to display an object information interface, wherein the object information interface includes at least one object;
an image acquiring module, configured to acquire a pathological image of a target object in response to a select operation on the target object on the object information interface;
a sub-image determining module, configured to determine at least one pathological sub-image of a target type from the pathological image; and
a sub-image displaying module, configured to display the at least one pathological sub-image of the target type on the object information interface.

In some embodiments, the sub-image displaying module includes:
a first displaying unit, configured to display the at least one pathological sub-image of the target type in an image display region of the object information interface; or
a second displaying unit, configured to display the pathological image on the object information interface, and display a frame of the at least one pathological sub-image in the pathological image.

In some embodiments, the first displaying unit is configured to:
in the case that the target type includes a plurality of sub-types, display a plurality of pathological sub-images in partitions according to different sub-types of the plurality of pathological sub-images;
display a plurality of pathological sub-images in an order of numbers of cells in the plurality of pathological sub-images;
display a plurality of pathological sub-images in an order of quality parameters of the plurality of pathological sub-images; or
display a plurality of pathological sub-images in an order of confidence levels of the plurality of pathological sub-images, wherein the confidence level of the pathological sub-image indicates a credibility that the pathological sub-image is of the target type.

In some embodiments, the target type includes a plurality of sub-types, a number of pathological sub-images of any of the plurality of sub-types in the image display region is not greater than a target number, and the first displaying unit is configured to:
cancel displaying of pathological sub-images of sub-types other than a first sub-type of the plurality of sub-types in the image display region in response to an expand operation on the first sub-type, and display each of pathological sub-images of the first sub-type.

In some embodiments, the apparatus further includes:
an zooming-in displaying module, configured to display a first pathological sub-image zoomed-in by a target magnification in response to a select operation on the displayed first pathological sub-image.

In some embodiments, the zooming-in displaying module is further configured to:
display image information of the first pathological sub-image in a first region in the zoomed-in first pathological sub-image, wherein the image information includes at least one of the target type of the first pathological sub-image and a confidence level of the first pathological sub-image.

In some embodiments, the zooming-in displaying module is further configured to:
display, in response to a display duration of the image information in the first region being equal to a target duration, the image information in a second region outside the zoomed-in first pathological sub-image,
wherein a size of the second region is less than a size of the first region.

In some embodiments, the zooming-in displaying module further includes:
a first zooming-in unit, configured to display the first pathological sub-image zoomed-in by the target magnification in response to a touch operation on the first pathological sub-image; or
a second zooming-in unit, configured to: in the case that the object information interface further includes an image identifier of the at least one pathological sub-image, display the first pathological sub-image zoomed-in by the target magnification in response to a touch operation on the image identifier of the at least one pathological sub-image.

In some embodiments, the sub-image displaying module includes:
a third displaying unit, configured to display the at least one pathological sub-image of the target type on the object information interface, and display a confidence level indicator of the at least one pathological sub-image, wherein the confidence level indicator of the at least one pathological sub-image indicates a confidence level of the at least one pathological sub-image, and the confidence level indicates a credibility that the pathological sub-image is of the target type.

In some embodiments, the sub-image determining module is configured to at least one of:
determine at least one pathological sub-image including abnormal cells from the pathological image;
determine at least one pathological sub-image including cells in a target state from the pathological image, wherein the target state includes a negative state and a positive state; and
determine at least one pathological sub-image that a quality parameter meets a target quality condition from the pathological image.

In some embodiments, the sub-image determining module includes:
an identifying unit, configured to acquire at least one position information by identifying the pathological image, wherein the position information indicates a region of the target type in the pathological image; and
a screening out unit, configured to screen out a region indicated by the at least one position information from the pathological image, and determine the screened-out region as the at least one pathological sub-image.

In some embodiments, the pathological image is a pathological image of liquid-based cytology or a pathological image of histopathology.

In another aspect, a terminal is provided. The terminal includes: a processor and a memory storing at least one computer program, wherein the processor, when loading and running the at least one computer program, is caused to perform the method for displaying images in the above aspect.

In another aspect, a computer-readable storage medium is provided. The computer-readable storage medium stores at least one computer program, wherein the at least one computer program, when loaded and run by a processor, causes the processor to perform the method for displaying images in the above aspect.

In another aspect, a computer program product is provided. The computer program product includes at least one computer program, wherein the at least one computer program, when loaded and run by a processor, causes the processor to perform the method for displaying images in the above aspect.

In the method and apparatus for displaying images, a terminal, and a storage medium in the embodiments of the present disclosure, at least one object is displayed on the object information interface, and the pathological image of the target object is acquired in response to the select operation on the target object. At least one pathological sub-image of a target type is determined from the pathological image and is displayed, such that key information with higher reference value in the pathological image is displayed, pertinence of displaying the pathological image is improved, and the display effect of the pathological image is greater.

### BRIEF DESCRIPTION OF DRAWINGS

FIG. 1 is a schematic diagram of an implementation environment according to some embodiments of the present disclosure;
FIG. 2 is a schematic diagram of an application scenario according to some embodiments of the present disclosure;
FIG. 3 is a schematic diagram of another application scenario according to some embodiments of the present disclosure;
FIG. 4 is a flowchart of a method for displaying images according to some embodiments of the present disclosure;
FIG. 5 is a flowchart of another method for displaying images according to some embodiments of the present disclosure;
FIG. 6 is a schematic diagram of an object information interface according to some embodiments of the present disclosure;
FIG. 7 is a flowchart of acquisition of a pathological image according to some embodiments of the present disclosure;
FIG. 8 is a schematic diagram of a manner of classifying pathological images according to some embodiments of the present disclosure;
FIG. 9 is a schematic diagram of another object information interface according to some embodiments of the present disclosure;
FIG. 10 is a schematic diagram of a pathological sub-image according to some embodiments of the present disclosure;
FIG. 11 is a schematic diagram of a method for displaying a pathological sub-image according to some embodiments of the present disclosure;
FIG. 12 is a flowchart of another method for displaying images according to some embodiments of the present disclosure;
FIG. 13 is a schematic diagram of another object information interface according to some embodiments of the present disclosure;
FIG. 14 is a schematic diagram of another object information interface according to some embodiments of the present disclosure;
FIG. 15 is a flowchart of another method for displaying images according to some embodiments of the present disclosure;
FIG. 16 is a schematic diagram of display of a pathological image according to some embodiments of the present disclosure;
FIG. 17 is a flowchart of another method for displaying images according to some embodiments of the present disclosure;
FIG. 18 is a schematic diagram of another object information interface according to some embodiments of the present disclosure;
FIG. 19 is a schematic diagram of another object information interface according to some embodiments of the present disclosure;
FIG. 20 is a schematic diagram of another object information interface according to some embodiments of the present disclosure;
FIG. 21 is a flowchart of another method for displaying images according to some embodiments of the present disclosure;
FIG. 22 is a schematic structural diagram of an apparatus for displaying images according to some embodiments of the present disclosure;
FIG. 23 is a schematic structural diagram of another apparatus for displaying images according to some embodiments of the present disclosure; and
FIG. 24 is a schematic structural diagram of a terminal according to some embodiments of the present disclosure.

### DETAILED DESCRIPTION

For clearer descriptions of the objectives, technical solutions, and advantages of the present disclosure, the embodiments of the present disclosure are described in detail hereinafter with reference to the accompanying drawings.

It should be understood that the terms "first," "second," and the like in the present disclosure are used to describe various concepts herein, and the concepts are not limited by the terms, unless specifically defined. The terms are only used to distinguish one concept from another concept. Illustratively, the first region is referred to as the second region without departing from the scope of the present disclosure, and the second region can be referred to as the first region similarly.

The term "at least one" refers to one or more. Illustratively, at least one pathological sub-image may be one pathological sub-image, two pathological sub-images, three pathological sub-images, or any integer number of pathological sub-images greater than one. The term "a plurality of" refers to two or more. Illustratively, a plurality of pathological sub-images refer to two pathological sub-images, three pathological sub-images, or any integer number of pathological sub-images greater than two. The term "each" refers to every one of at least one. Illustratively, each pathological sub-image refers to every one of a plurality of pathological sub-images. In the case that a plurality of pathological sub-images include three pathological sub-images, each of the plurality of pathological sub-images refers to every one of the three pathological sub-images.

It should be noted that the user information (including, but not limited to object information, pathological images, and the like) involved in the present disclosure is authorized by the user or sufficiently authorized by the parties.

The method for displaying images in the embodiments of the present disclosure is performed by a terminal. The terminal displays at least one object on an object information interface. In the case that the user selects an object, the terminal acquires a pathological image of the object, and displays at least one pathological sub-image of the target type in the pathological image on the object information interface, such that key information with higher reference value in the pathological image is displayed, and the pertinence of displaying the pathological image is improved.

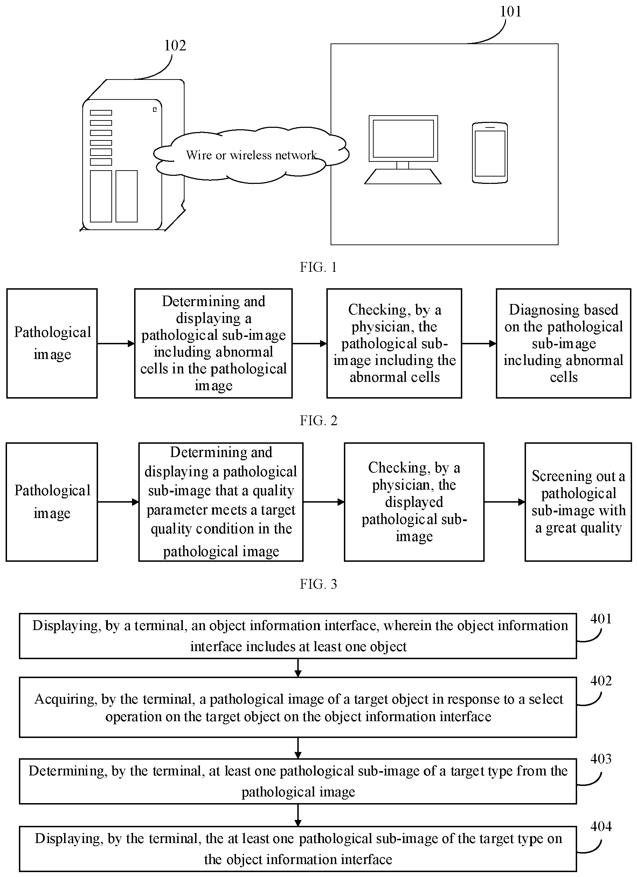

In some embodiments of the present disclosure, an implementation environment of the method for displaying images is provided. FIG. 1 is a schematic diagram of an implementation environment according to some embodiments of the present disclosure. As shown in FIG. 1, the implementation environment includes a terminal 101 and a server 102. The terminal 101 and the server 102 are connected over a wireless or wire network.

The terminal 101 is configured to request the server 102 to identify the pathological image, and the server 102 is configured to identify a pathological sub-image of a target type from the pathological image and send the identified pathological sub-image to the terminal 101. The terminal 101 is configured to display the pathological sub-image of the target type to the user.

In some embodiments, the terminal 101 is disposed with a target client served by the server 102, and the terminal 101 achieves functions, such as image identification, data transmission, message interaction, and the like through the target client. In some embodiments, the target client is a client in an operation system of the terminal 101 or a client provided by a third party. In some embodiments, the server 102 is a backend terminal of the target client or a cloud server for providing cloud computation, cloud storage, and other services.

In some embodiments, the terminal 101 is a smart mobile phone, a tablet computer, a laptop, a desktop computer, a smart speaker, a smart watch, a smart voice interaction device, a smart home appliance, and the like, and the server 102 is one server, a server cluster composed of several servers, or a cloud computing service center.

The method for displaying images in the embodiments of the present disclosure is applicable to any scenario of displaying the pathological image.

Illustratively, the method is applicable to a scenario of assisting the doctor for the auxiliary diagnosis. In such scenario, a pathological sub-image of a target type is a pathological sub-image including abnormal cells. The terminal displays patient information of several patients on a patient information interface. In the case that a physician wants to diagnose a patient, a select operation on the patient is performed on the patient information interface. As shown in FIG. 2, the terminal acquires a pathological image of the patient, and determines and displays a pathological sub-image including abnormal cells in the pathological image, such that the physician diagnoses the condition of the patient by viewing the pathological sub-image displayed on the terminal and combining other related information of the patient. Thus, in the method for displaying images in the embodiments of the present disclosure, the physician can directly view the pathological sub-image including abnormal cells without analyzing and researching the whole pathological image of the patient, such that the pertinence of displaying the pathological image is improved, and the physician can quickly diagnose.

Illustratively, the method is applicable to a scenario of managing the pathological image. In such scenario, a pathological sub-image of a target type is a pathological sub-image that a quality parameter meets a target quality condition. Image qualities of different regions in one pathological image are different, and the image quality of the pathological image is controlled and screened by mutually viewing the whole pathological image in some practices. While in the method for displaying images in the embodiments of the present disclosure, in the case that the user wants to view a pathological image of an object, the user performs a select operation on the object. As shown in FIG. 3, the terminal acquires the pathological image of the object, and determines and displays the pathological sub-image that the quality parameter meets the target quality condition in the pathological image, such that the pathological sub-images with the greater qualities are selected, and pathological sub-images with less qualities are filtered. Thus, the user is convenient to view and analyze, the image quality is automatically controlled and screened, and the mutual cost is reduced.

FIG. 4 is a flowchart of a method for displaying images according to some embodiments of the present disclosure. An execution body of the embodiments of the present disclosure is the terminal. Referring to FIG. 4, the method includes the following processes.

In S401, the terminal displays an object information interface, wherein the object information interface includes at least one object.

In some embodiments, the object is a person, an animal, a plant, and the like, which is not limited in the embodiments of the present disclosure. The object information interface is configured to display information of the at least one object. For example, the object information interface is divided into a plurality of different regions, and each region is configured to display information of different types.

In S402, the terminal acquires a pathological image of a target object in response to a select operation on the target object on the object information interface.

The target object on the object information interface is any one of the at least one object. The select operation on the target object is configured to request display of the pathological image of the target object. Thus, upon viewing the objects on the object information interface, the user performs the select operation on the target object in the case of wanting to view the pathological image of the target object, and the terminal acquires the pathological image of the target object in response to the select operation on the target object.

The pathological image is also referred to as a pathological section image, a digital section image, and the like, and the pathological image of the target object is configured to display pathological morphology images of body organs, tissues or cells of the object. For example, the pathological image is a whole slide image (WSI) that is an image acquired by scanning the pathological micro-section by a digital pathological scanner, and the digital pathological scanner is composed of an optical system, a linear scan camera, and the like.

In some embodiments, the pathological image is a pathological image of liquid-based cytology or a pathological image of histopathology, or other types of pathological images, which is not limited in the embodiments of the present disclosure.

In S403, the terminal determines at least one pathological sub-image of a target type from the pathological image.

The terminal identifies the pathological image, and determines at least one pathological sub-image of the target type from the pathological image. The pathological sub-image is a region in the pathological image. That is, the terminal identifies a region of the target type from the pathological image, screens out the identified region, and determines the screened-out region as the pathological sub-image. The pathological sub-image of the target type includes key information with the reference value, that is, the information of interest to the user.

The pathological sub-images in the pathological image are classified in different classification standards, and target types are diversified accordingly. The terminal determines different pathological sub-images of different target types in different identification manners, which are not limited in the embodiments of the present disclosure.

In S404, the terminal displays the at least one pathological sub-image of the target type on the object information interface.

Upon determining the at least one pathological sub-image of the target type, the terminal displays the at least one pathological sub-image on the object information interface for viewing of the user. Thus, the user is capable of viewing the pathological sub-image of the target type displayed by the terminal without viewing the whole pathological image and looking up the pathological sub-image of the target type.

In the method in the embodiments of the present disclosure, at least one object is displayed on the object information interface, and the pathological image of the target object is acquired in response to the select operation on the target object. At least one pathological sub-image of a target type is determined from the pathological image and is displayed, such that key information with higher reference value in the pathological image is displayed, the pertinence of displaying the pathological image is improved, and the display effect of the pathological image is greater.

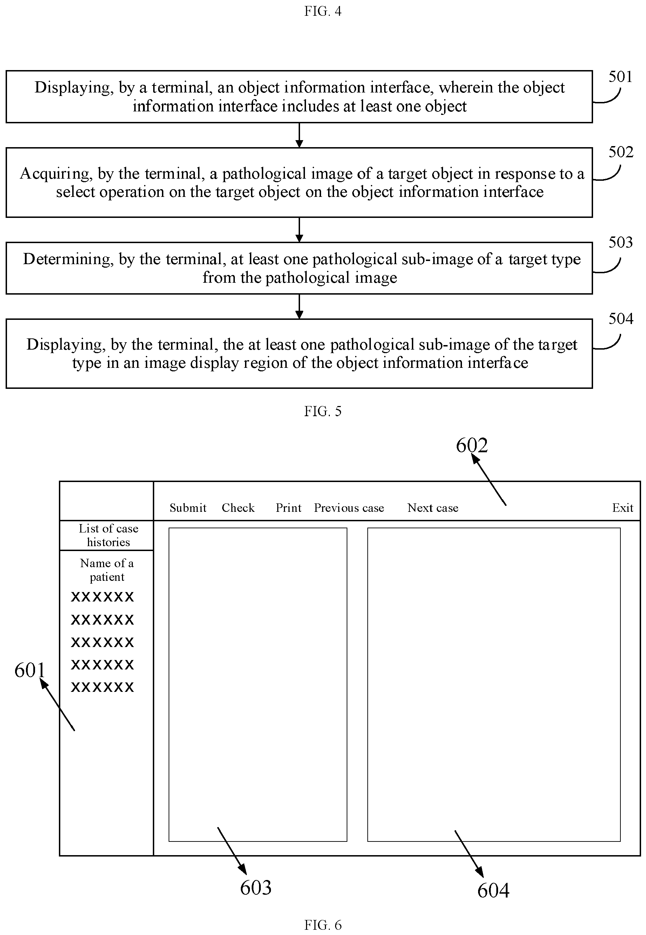

The processes of displaying the pathological image and the pathological sub-image are briefly described in the embodiments of FIG. 2. In some embodiments, upon determining the pathological sub-image of the target type, the terminal displays the pathological sub-image of the target type in the image display region of the object information interface, such that the user can quickly notices the pathological sub-images. The detailed processes can be referred to the following embodiments of FIG. 5. FIG. 5 is a flowchart of another method for displaying images according to some embodiments of the present disclosure. An execution body of the embodiments of the present disclosure is the terminal, and the embodiments of the present disclosure are described by taking the pathological sub-image being displayed in the image display region of the object information interface as an example. Referring to FIG. 5, the method includes the following processes.

In S501, the terminal displays an object information interface, wherein the object information interface includes at least one object.

In some embodiments, the object is a person, an animal, a plant, and the like, which is not limited in the embodiments of the present disclosure. The object information interface is configured to display information of the at least one object.

For example, the object is the user, the object information interface is a user information interface, and the user information interface displays the name, age, photo, contact information, pathological image, medical record report of the user, and the like, which is not limited in the embodiments of the present disclosure.

FIG. 6 is a schematic diagram of an object information interface according to some embodiments of the present disclosure. The object information interface is a patient information interface in the medical field to record the information of the patient. As shown in FIG. 6, the patient information interface includes four regions, that is, a region 601, a region 602, a region 603, and a region 604. The region 601 is configured to display identifiers of at least one patient, for example, the name or number of the patient, and the like. The case report or the pathological image of the patient is viewed by taping the identifier of the patient in the region 601. The region 602 is a "command bar" on the patient information interface, and includes a plurality of touch options. Operations, such as submitting the case report, checking the case report, printing the case report, viewing the previous case, viewing the next case, exiting the patient information interface, and the like are performed in the region 602. The region 603 is configured to display the case report of the patient, and operations, such as filling, modifying, and viewing the case report of the patient are performed in the region 603. The region 604 is configured to display the pathological sub-image of the target type in the pathological image of the patient.

In S502, the terminal acquires a pathological image of a target object in response to a select operation on the target object on the object information interface.

The target object on the object information interface is any one of the at least one object. The select operation on the target object is configured to request the display of the pathological image of the target object. Thus, upon viewing the objects on the object information interface, the user performs the select operation on the target object in the case of wanting to view the pathological image of the target object, and the terminal acquires the pathological image of the target object in response to the select operation on the target object.

In some embodiments, the terminal stores the pathological images of the objects displayed on the object information interface, and the pathological image of any object is correspondingly stored with the object identifier of the object. The terminal looks up, in response to the select operation on the target object, the pathological image corresponding to the object identifier of the target object from the stored pathological images, and acquires the pathological image. The pathological image is the pathological image of the target object. In some embodiments, the server stores the pathological images of the objects, and the pathological image of any object is correspondingly stored with the object identifier of the object. The terminal sends, in response to the select operation on the target object, an image acquire request carrying the object identifier of the target object to the server. The server looks up, in response to the image acquire request, the pathological image corresponding to the object identifier of the target object from the stored pathological images, acquires the pathological image, and sends the pathological image to the terminal. The terminal receives the pathological image. The pathological image is the pathological image of the target object.

In some embodiments, the terminal acquires a pathological file of the target object in response to the select operation on the target object on the object information interface, and acquires the pathological image in the pathological file by parsing the pathological file.

The pathological file is a pathological image stored in a file form. The pathological file of the target object includes a plurality of pathological images with different zoom levels. That is, the contents of the plurality of pathological images in the pathological file are the same, and the sizes of the plurality of pathological images are different. Parsing the pathological file indicates that the pathological images in the pathological file are acquired in a structured form, such that the subsequent processes for the pathological images are convenient. For example, the terminal acquires the pathological image with a target zoom level, and S503 to S504 are performed based on the acquired pathological image. In some embodiments, the terminal parses the pathological file by a software development kit matched with the format of the pathological file, or parses the pathological file based on a file configuration corresponding to the pathological file. In some embodiments, the pathological file is a file with a svs format (a format of the file), and the terminal parses the pathological file by OpenSlide.

In some embodiments, upon acquiring the pathological file, the terminal checks the pathological file and acquires the check result. The check result indicates whether the pathological file is damaged. In the case that the pathological file is not damaged, the pathological file is parses to acquire the pathological image.

As the pathological file may be damaged in the storage process or transmission process of the pathological file, the terminal checks whether the pathological file is damaged prior to parsing the pathological file, such that whether the pathological file is damaged is determined. In the case that the check result indicates that the pathological file is not damaged, the pathological image can be successfully parsed from the pathological file, and the terminal parses the pathological file. In the case that the check result indicates that the pathological file is damaged, the pathological image cannot be parsed from the pathological file or the parsed pathological image is error, and the terminal does not require to parse the pathological file. In some embodiments, the terminal checks the pathological file in md5 (a message digest algorithm), file flag bits, and other manners.

In some embodiments, the terminal stores the pathological files of the objects displayed on the object information interface, and the pathological file of any object is correspondingly stored with the object identifier of the object. The terminal looks up, in response to the select operation on the target object, the pathological file corresponding to the object identifier of the target object from the stored pathological files, and acquires the pathological file. The pathological file is a pathological file of the target object. In some embodiments, the server stores the pathological files of the objects, and the pathological file of any object is correspondingly stored with the object identifier of the object. The terminal sends, in response to the select operation on the target object, an image acquire request carrying the object identifier of the target object to the server. The server looks up, in response to the image acquire request, the pathological file corresponding to the object identifier of the target object from the stored pathological files, acquires the pathological file, and sends the pathological file to the terminal. The terminal receives the pathological file. The pathological file is a pathological file of the target object.

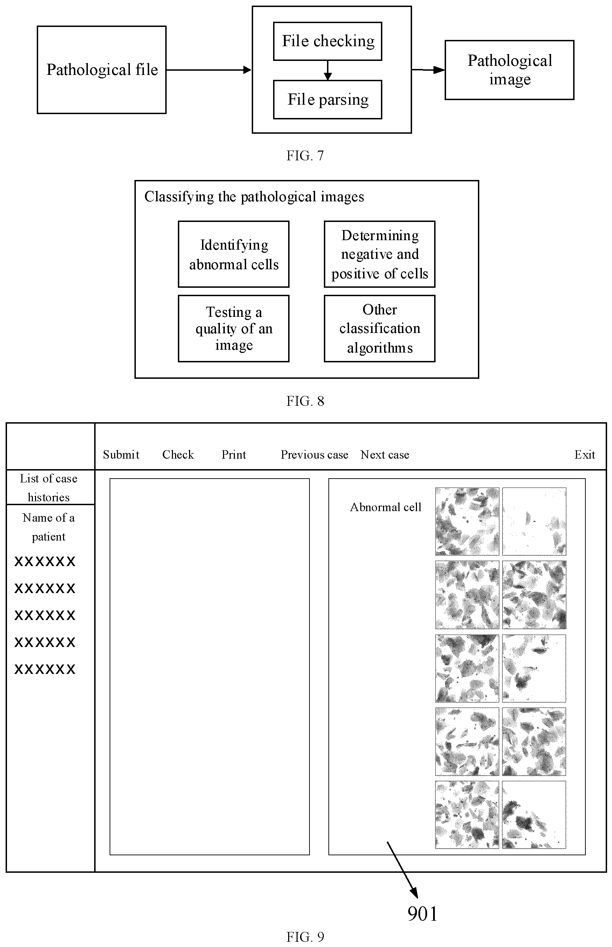

FIG. 7 is a flowchart of acquisition of a pathological image according to some embodiments of the present disclosure. As shown in FIG. 7, the process of acquiring the pathological image from the pathological file includes two stages, that is, a file checking stage and a file parsing stage. The terminal first checks the pathological file, and parses the pathological file in the case that the pathological file is successfully checked, such that the pathological image in the pathological file is acquired.

In the embodiments of the present disclosure, by checking the pathological file, the validity of the pathological file is ensured, such that the accuracy of the pathological image acquired by parsing the pathological file is ensured, and the parsed error pathological image is avoided being processed in the case that the pathological file is damaged.

In S503, the terminal determines at least one pathological sub-image of a target type from the pathological image.

The terminal identifies the pathological image upon acquiring the pathological image, and determines at least one pathological sub-image of the target type from the pathological image. The pathological sub-image of the target type includes key information with the reference value, that is, the information of interest to the user.

In some embodiments, the terminal determines at least one pathological sub-image of a target type from the pathological image in at least one of the following manners.

In a first determining manner, the terminal determines at least one pathological sub-image including abnormal cells from the pathological image.

The terminal identifies the abnormal cells from the pathological image, and determines at least one pathological sub-image including abnormal cells from the pathological image based on the identification result. In the medical field, whether the body of the patient includes the abnormal cells, the type of the abnormal cells, and a lesion degree of the abnormal cell are key information used to assist doctors in disease diagnosis. Thus, the terminal can determine at least one pathological sub-image including abnormal cells and display to the physician, such that the physician can quickly diagnose.

The abnormal cell is a cell that is abnormal. The abnormality of the cell includes a plurality of case, for example, glandular epithelial abnormalities, high-grade squamous cell lesions, low-grade squamous cell lesions, and other abnormalities.

In some embodiments, the terminal identifies the pathological image by an abnormal cell identification and classification algorithm to acquire at least one pathological sub-image including abnormal cells.

In a second determining manner, the terminal determines at least one pathological sub-image including cells in a target state from the pathological image. The target state includes a negative state and a positive state.

The terminal identifies the cells in the target state, and determines at least one pathological sub-image including cells in the target state from the pathological image based on the identification result. In the medical field, the cells in the body of the patient being the negative state or the positive state is key information used to assist doctors in disease diagnosis. Thus, the terminal can determine the pathological sub-image including cells in the negative state or the positive state.

In some embodiments, the terminal identifies the pathological image by a section negative and positive determination algorithm to acquire at least one pathological sub-image including cells in the target state.

In a third determining manner, the terminal determines at least one pathological sub-image that a quality parameter meets a target quality condition from the pathological image.

The terminal tests the quality of the pathological image, and determines, based on the test result, at least one pathological sub-image that the quality parameter meets the target quality condition from the pathological image.

In some embodiments, the quality parameter indicates the quality of the pathological sub-image. For example, the greater the quality parameter, the great the quality of the pathological sub-image. In some embodiments, the target quality condition is that the quality parameter is less than a first threshold, such that the pathological sub-image with the less quality is screened. In some embodiments, the target quality condition is that the quality parameter is greater than a second threshold, such that the pathological sub-image with the great quality is screened. The first threshold is less than the second threshold.

In some embodiments, the quality parameter indicates whether the pathological sub-image has the quality problem and the type of the quality problem. For example, the quality parameter being 1 indicates that the pathological sub-image is blurred, the quality parameter being 2 indicates that the pathological sub-image is incoherently spliced, the quality parameter being 3 indicates that the pathological sub-image is blocked by a foreign matter, the quality parameter being 4 indicates that the pathological sub-image does not have the quality problem, and the like. Correspondingly, the target quality condition is that the quality parameter is 4, the quality parameter is less than 4, or the quality parameter is 1, and the like. Different types of the pathological sub-images are screened out based on the quality of the image by different target quality conditions, such that the quality of the pathological sub-image is controlled.

FIG. 8 is a schematic diagram of a manner of classifying pathological images according to some embodiments of the present disclosure. As shown in FIG. 8, the pathological images are classified in at least three manners, that is, identifying the abnormal cells, determining the negative state or the positive state of the cell, and testing the quality of the image. It should be noted that the embodiments of the present disclosure are described by taking the above three manners as an example. In addition, the terminal classifies the pathological images by other classification algorithms to determine the pathological sub-images of the target type, which is not limited in the embodiments of the present disclosure.

In some embodiments, the terminal identifies the pathological image to acquire at least one region of the target type in the pathological image, and the at least one region is screened out from the pathological image to acquire at least one pathological sub-image of the target type. In some embodiments, pixel points in the pathological sub-image of the target type have specific features. The terminal performs the feature extraction on the pathological image to acquire the features of the pixel points in the pathological image, and thus determines at least one region of the target type based on the features of the pixel points.

In some embodiments, the terminal identifies the pathological image to acquire at least one position information, and the position information indicates a region of the target type in the pathological image. The terminal screens out the region indicated by the at least one position information, and determines the screened-out region as the pathological sub-image.

The terminal identifies the pathological image to acquire the position information, and positions the region of the target type in the image based on the position information. The region indicated by the position information is in a rectangular shape, a circle shape, or other shapes. As it is not convenient to display the position information to the user, the terminal screens out the pathological sub-image of the target type from the pathological image based on the position information, and subsequently displays the pathological sub-image to the user for viewing of the user.

In some embodiments, the terminal identifies the pathological image to acquire the position information and corresponding type information. The type information is configured to indicate the target type, and the type of the region indicated by the position information is the target type indicated by the type information. The terminal stores the position information and the type information correspondingly, subsequently screens out the pathological sub-image from the pathological image based on the position information, and determines, based on the type information corresponding to the position information, that the type of the pathological sub-image is the target type indicated by the type information.

In S504, the terminal displays the at least one pathological sub-image of the target type in the image display region of the object information interface.

Upon determining the at least one pathological sub-image of the target type, the terminal displays the at least one pathological sub-image in the image display region of the object information interface, and thus the user is capable of viewing the pathological sub-image in the image display region without viewing the whole pathological image and looking up the pathological sub-image of the target type.

FIG. 9 is a schematic diagram of another object information interface according to some embodiments of the present disclosure. As shown in FIG. 9, the object information interface is the same as that shown in FIG. 6. The object information interface includes an image display region 901, and the terminal displays a plurality of pathological sub-images in the image display region 901 upon acquiring the plurality of pathological sub-images including abnormal cells.

In some embodiments, the terminal displays the at least one pathological sub-image of the target type in the image display region of the object information interface in the four display manners.

In a first display manner, the target type includes a plurality of sub-types, and the terminal displays a plurality of pathological sub-images in partitions according to different sub-types of the plurality of pathological sub-images.

A plurality of pathological sub-images of the target type include pathological sub-images of any sub-type. Thus, in the plurality of pathological sub-images determined in S503, sub-types of any two pathological sub-images are the same or different, and the plurality of pathological sub-images are displayed in partitions in the image display region according to different sub-types of the plurality of pathological sub-images.

That is, the terminal divides the image display region to a plurality of sub-regions, and each sub-type corresponds to one sub-region. The terminal displays at least one pathological sub-image of the sub-type on the sub-region corresponding to the sub-type. In the embodiments of the present disclosure, the plurality of pathological sub-images of different sub-types are displayed in partitions, such that the user can conveniently and overall view pathological sub-images of the same sub-type, and the display effect of the pathological sub-images is improved.

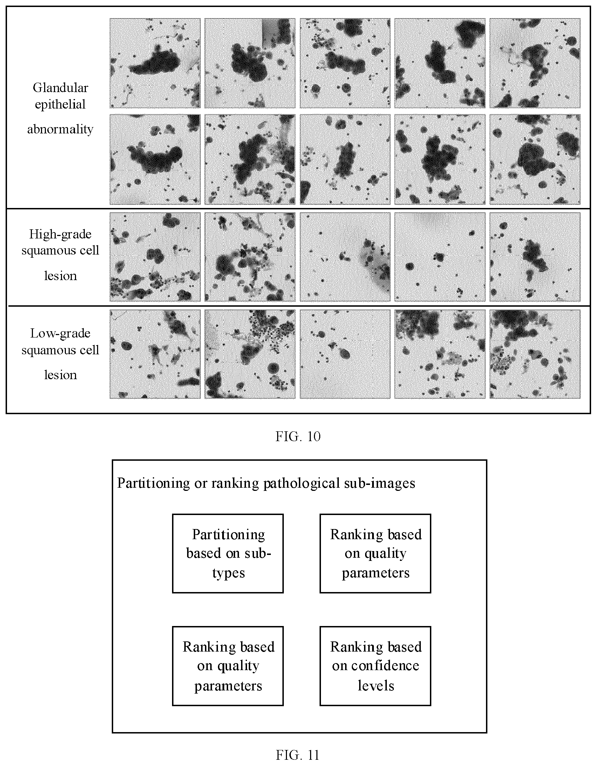

For example, the target type is that the pathological sub-image includes the abnormal cells, and the target type includes a plurality of sub-types, that is, glandular epithelial abnormalities, high-grade squamous cell lesions, and low-grade squamous cell lesions. Thus, as shown in FIG. 10, the terminal displays the plurality of pathological sub-images including the abnormal cells in partitions in an order of the glandular epithelial abnormalities, high-grade squamous cell lesions, and low-grade squamous cell lesions.

In a second display manner, the terminal displays a plurality of pathological sub-images in an order of numbers of cells in the plurality of pathological sub-images.

The terminal determines the number of the cells in each pathological sub-image, and displays the plurality of pathological sub-images in the order of the numbers of the cells in the plurality of pathological sub-images. For example, the terminal displays the plurality of pathological sub-images in a descending order of the numbers of the cells, such that the pathological sub-image including the greater number of the cells is first displayed. In some embodiments, the terminal displays the plurality of pathological sub-images in an ascending order of the numbers of the cells, which is not limited in the embodiments of the present disclosure.

In a third display manner, the terminal displays a plurality of pathological sub-images in an order of quality parameters of the plurality of pathological sub-images.

The terminal determines the quality parameters of the plurality of pathological sub-images, and the quality parameter indicates the quality of the pathological sub-image. For example, the greater the quality parameter, the greater the quality of the pathological sub-image. The terminal displays the plurality of pathological sub-images in the order of quality parameters of the plurality of pathological sub-images. For example, the terminal displays the plurality of pathological sub-images in a descending order of quality parameters, such that the clear pathological sub-image is first displayed. In some embodiments, the terminal displays the plurality of pathological sub-images in an ascending order of quality parameters, which is not limited in the embodiments of the present disclosure.

In a fourth display manner, the terminal displays a plurality of pathological sub-images in an order of confidence levels of the plurality of pathological sub-images.

The terminal determines confidence levels of the plurality of pathological sub-images, and the confidence level of the pathological sub-image indicates a credibility that the pathological sub-image is of the target type, that is, the possibility of the pathological sub-image being of the target type. For example, the greater the confidence level, the greater the credibility that the pathological sub-image is of the target type. The terminal displays the plurality of pathological sub-images in the order of the confidence levels of the plurality of pathological sub-images. For example, the terminal displays the plurality of pathological sub-images in a descending order of the confidence levels, such that the pathological sub-image that the credibility of being the target type is great is first displayed. In some embodiments, the terminal displays the plurality of pathological sub-images in an ascending order of the confidence levels, which is not limited in the embodiments of the present disclosure.

In the embodiments of the present disclosure, the plurality of pathological sub-images are displayed in the order of the numbers of the cells, the quality parameters, or the confidence levels, such that the user directly view in the order of the features. Thus, key images in the plurality of pathological sub-images are quickly found, and the display effect of the pathological sub-images is further improved.

FIG. 11 is a schematic diagram of a method for displaying a pathological sub-image according to some embodiments of the present disclosure. As shown in FIG. 11, the pathological sub-images are displayed in four display manners, that is, partition display according to the sub-regions, display in the order of the quality parameters, display in the order of the numbers of the cells, and display in the order of the confidence levels. It should be noted that the embodiments of the present disclosure are described in the above four manners, and the terminal may also display in other partition manners or other orders, which is not limited in the embodiments of the present disclosure.

It should be noted that the embodiments of the present disclosure are described by taking the display of the pathological sub-images in the image display region as an example, and at least one pathological sub-image of the target type is displayed on the object display region in other manners.

In the method in the embodiments of the present disclosure, at least one object is displayed on the object information interface, and the pathological image of the target object is acquired in response to the select operation on the target object. At least one pathological sub-image of a target type is determined from the pathological image and is displayed, such that key information with higher reference value in the pathological image is displayed, the pertinence of displaying the pathological image is improved, and the display effect of the pathological image is greater.

In addition, the pathological sub-images of different sub-types are displayed in partitions, such that the user can conveniently and overall view pathological sub-images of the same sub-type, and the display effect of the pathological sub-images is improved.

In addition, the plurality of pathological sub-images are displayed in the order of the numbers of the cells, the quality parameters, or the confidence levels, such that the user directly view in the order of the features. Thus, key images in the plurality of pathological sub-images are quickly found, and the display effect of the pathological sub-images is further improved.

In addition, by checking the pathological file, the validity of the pathological file is ensured, such that the accuracy of the pathological image acquired by parsing the pathological file is ensured, and the parsed error pathological image is avoided being processed in the case that the pathological file is damaged.

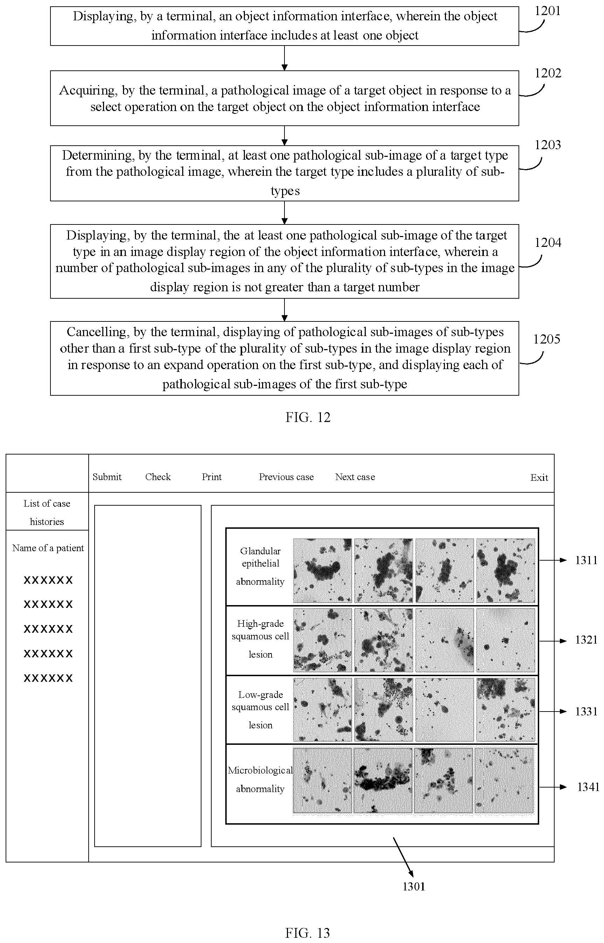

In some embodiments, on the basis of the embodiments of FIG. 5, the target type includes a plurality of sub-types, and the terminal displays a limited number of pathological sub-images of each sub-type, expands and displays each pathological sub-images of one sub-type based on an expand operation, and cancels displaying of the pathological sub-images of another sub-types. The detailed processes are referred to the following embodiments of FIG. 12. FIG. 12 is a flowchart of another method for displaying images according to some embodiments of the present disclosure. An execution body of the embodiments of the present disclosure is the terminal, and the embodiments of the present disclosure are described by taking the target type including a plurality of sub-types as an example. Referring to FIG. 12, the method includes the following processes.

In S1201, the terminal displays an object information interface, wherein the object information interface includes at least one object.

In S1202, the terminal acquires a pathological image of a target object in response to a select operation on the target object on the object information interface.

The processes of S1201 and S1202 are the same as the processes of S501 to S502, which is not repeated herein.

In S1203, the terminal determines at least one pathological sub-image of a target type from the pathological image, wherein the target type includes a plurality of sub-types.

The processes of S1203 and the S503 are similar and differ in that the target type includes a plurality of sub-types.

In some embodiments, the target type is that the pathological sub-image is abnormal. The target type includes a plurality of sub-types according to different type of abnormalities, for example, the sub-type is the glandular epithelial abnormality, the high-grade squamous cell lesion, the low-grade squamous cell lesion, and the like.

In some embodiments, the target type is that the pathological sub-image has the quality problem. The target type includes a plurality of sub-types according to different type of the quality problems, for example, the sub-type is that the blur image, the incoherently spliced image, the foreign matter in the image, the mutilated image, and the like.

In S1204, the terminal displays the at least one pathological sub-image of the target type in the image display region of the object information interface, wherein a number of pathological sub-images in any of the plurality of sub-types in the image display region is not greater than a target number.

The processes of S1204 and the S504 are similar and differ in that the number of pathological sub-images in any of the plurality of sub-types in the image display region is not greater than a target number. That is, for each sub-type, only a limited number of pathological sub-images are displayed in the image display region. In some embodiments, the target number is a predetermined number, for example, 6, 8, and the like.

FIG. 13 is a schematic diagram of another object information interface according to some embodiments of the present disclosure. As shown in FIG. 13, the object information interface is the same as the object information interface shown in FIG. 6. The object information interface includes an image display region 1301, and the image display region 1301 is divided to a plurality of sub-regions, that is, a sub-region 1311 corresponding to the glandular epithelial abnormality, a sub-region 1321 corresponding to the high-grade squamous cell lesion, a sub-region 1331 corresponding to the low-grade squamous cell lesion, and a sub-region 1341 corresponding to microbiological abnormality. Each sub-region only displays four pathological sub-images of the corresponding sub-type.

In S1205, the terminal cancels displaying of pathological sub-images of sub-types other than a first sub-type of the plurality of sub-types in the image display region in response to an expand operation on the first sub-type, and displays each of pathological sub-images of the first sub-type.

The first sub-type is any of the plurality of sub-types, and the expand operation on the first sub-type is configured to request display of the pathological sub-images of the sub-type. Thus, in the case that the user wants to view other pathological sub-images of the first sub-type, the user performs the expand operation on the first sub-type, and the terminal cancels the displaying of pathological sub-images of sub-types other than the first sub-type of the plurality of sub-types in the image display region in response to the expand operation on the first sub-type, and displays each of pathological sub-images of the first sub-type.

In the embodiments of the present disclosure, a limited number of pathological sub-images of each sub-type are displayed in the image display region. In the case that the expand operation on any sub-type is performed, the displaying of the pathological sub-images of other sub-types in the image display region is cancelled, and the pathological sub-images of the sub-type are displayed, such that the flexibility of display of the pathological sub-images is improved.

In some embodiments, type identifiers of the sub-types are displayed in the image display region, and the terminal cancels displaying of pathological sub-images of sub-types other than the first sub-type of the plurality of sub-types in the image display region in response to a trigger operation on the type identifier corresponding to the first sub-type, and displays each of pathological sub-images of the first sub-type. For example, the trigger operation is a tap operation, a drag operation, and the like.

In some embodiments, expand options of the sub-types are displayed in the image display region, and the terminal cancels displaying of pathological sub-images of sub-types other than the first sub-type of the plurality of sub-types in the image display region in response to a trigger operation on the expand option corresponding to the first sub-type, and displays each of pathological sub-images of the first sub-type.

In some embodiments, the terminal cancels displaying of pathological sub-images of sub-types other than the first sub-type of the plurality of sub-types in the image display region in response to the expand option corresponding to the first sub-type, and displays a scroll list corresponding to the first sub-type. The scroll list includes the pathological sub-images of the first sub-type.

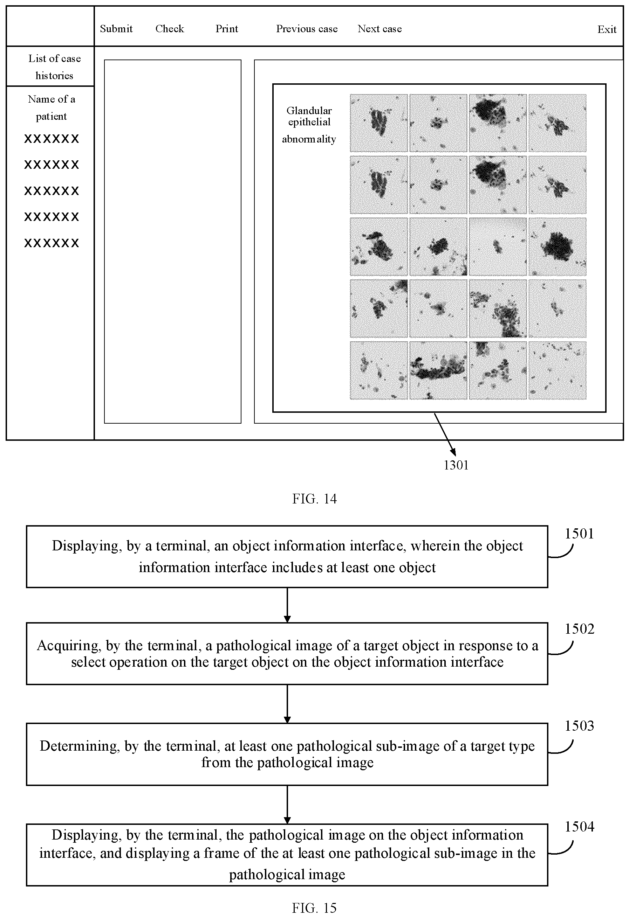

For example, as shown in FIG. 13, in the case that the user wants to view a plurality of pathological sub-images of the glandular epithelial abnormality, an expand operation on the "glandular epithelial abnormality" is performed on the object information interface shown in FIG. 13, and the terminal displays the object information interface shown in FIG. 14 in response to the expand operation. Referring to FIG. 14, the image display region 1301 does not include the pathological sub-images of the high-grade squamous cell lesion, the pathological sub-images of the low-grade squamous cell lesion, and the pathological sub-images of microbiological abnormality, and only includes the pathological sub-images of the glandular epithelial abnormality.

It should be noted that the embodiments of the present disclosure are described by taking the display of the pathological sub-images in the image display region as an example, and at least one pathological sub-image of the target type is displayed on the object display region in other manners.

In the method in the embodiments of the present disclosure, at least one object is displayed on the object information interface, and the pathological image of the target object is acquired in response to the select operation on the target object. At least one pathological sub-image of a target type is determined from the pathological image and is displayed, such that key information with higher reference value in the pathological image is displayed, the pertinence of displaying the pathological image is improved, and the display effect of the pathological image is greater.

In addition, a limited number of pathological sub-images of each sub-type are displayed in the image display region. In the case that the expand operation on any sub-type is performed, the displaying of the pathological sub-images of other sub-types in the image display region is cancelled, and the pathological sub-images of the sub-type are displayed, such that the flexibility of display of the pathological sub-images is improved.

The embodiments of FIG. 5 and FIG. 12 are described by taking the pathological sub-images of the target type being displayed in the image display region as an example. In some embodiments, the terminal displays a complete pathological image, frames of the pathological sub-images of the target type are displayed in the pathological image, and the detailed descriptions are referred to the following embodiments of FIG. 15. FIG. 15 is a flowchart of another method for displaying images according to some embodiments of the present disclosure. An execution body of the embodiments of the present disclosure is the terminal, and the embodiments of the present disclosure are described by taking the target type including a plurality of sub-types as an example. Referring to FIG. 15, the method includes the following processes.

In S1501, the terminal displays an object information interface, wherein the object information interface includes at least one object.

In S1502, the terminal acquires a pathological image of a target object in response to a select operation on the target object on the object information interface.

In S1503, the terminal determines at least one pathological sub-image of a target type from the pathological image.

The processes of S1501 and S1503 are the same as the processes of S501 to S503, which is not repeated herein.

In S1504, the terminal displays the pathological image on the object information interface, and displays a frame of the at least one pathological sub-image in the pathological image.

As the user may want to view other regions in the pathological image than the at least one pathological sub-image, the terminal directly displays the pathological image on the object information interface. The terminal displays the frame of the at least one pathological sub-image in the pathological image, such that the user can quickly view the at least one pathological sub-image. The frame functions as the mark and reminding. Thus, the pathological image is completely displayed, the pertinence of displaying the pathological image is ensured, and the overall display effect is improved.

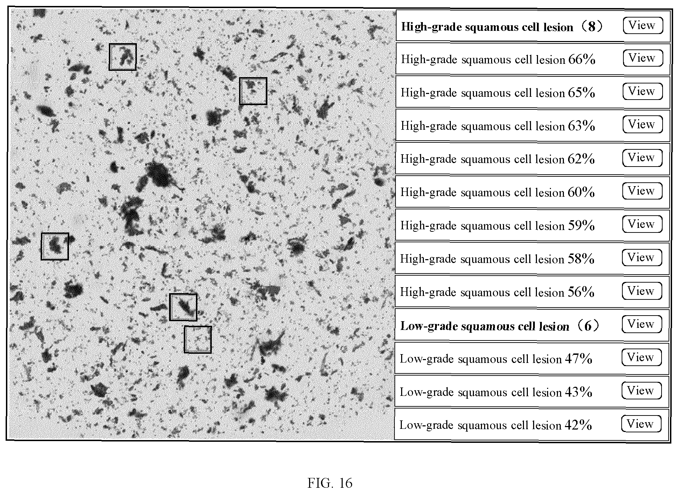

FIG. 16 is a schematic diagram of display of a pathological image according to some embodiments of the present disclosure. As shown in FIG. 16, the frames of a plurality of pathological sub-images are displayed in the pathological image on the object information interface. In addition, the image information of the pathological sub-images is displayed on the object information interface, and the image information includes the target type and the confidence level of the pathological sub-image. For example, it can be seen from FIG. 16 that the pathological image includes eight pathological sub-images of the high-grade squamous cell lesion, the confidence levels of the eight pathological sub-images are 66%, 65%, 63%, 62%, 60%, 59%, 58%, and 56%, the pathological image further includes six pathological sub-images of the low-grade squamous cell lesion, image information of three pathological sub-images of the low-grade squamous cell lesion is displayed on the object information interface, and the confidence levels of the three pathological sub-images are 47%, 43%, and 42%.

In the method in the embodiments of the present disclosure, at least one object is displayed on the object information interface, and the pathological image of the target object is acquired in response to the select operation on the target object. At least one pathological sub-image of a target type is determined from the pathological image and is displayed, such that key information with higher reference value in the pathological image is displayed, the pertinence of displaying the pathological image is improved, and the display effect of the pathological image is greater.

In addition, the pathological image is displayed on the object information interface, the frame of the at least one pathological sub-image is displayed in the pathological image, and the frame functions as the mark and reminding, such that the pathological image is completely displayed, the pertinence of displaying the pathological image is ensured, and the overall display effect is improved.

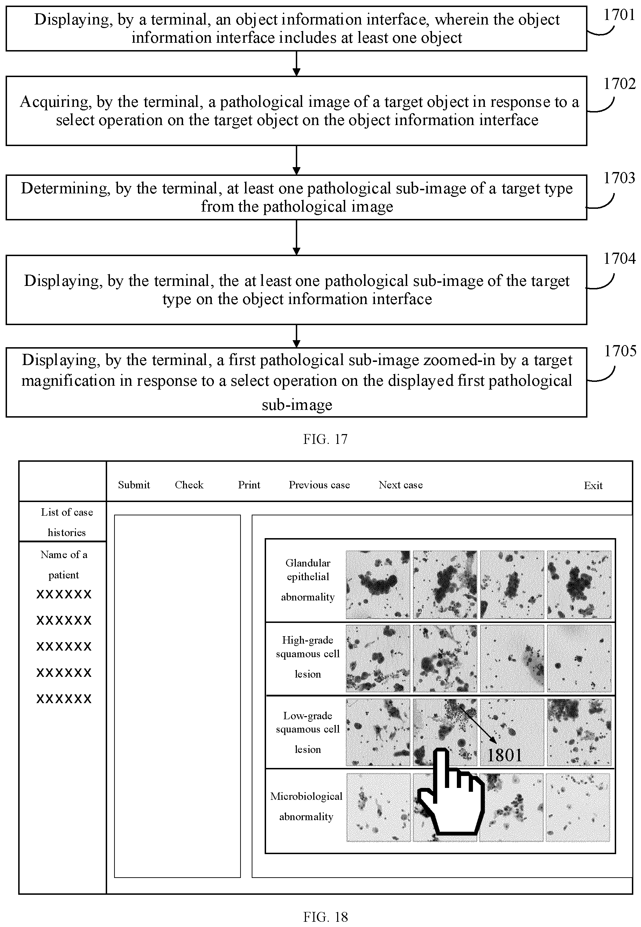

In some embodiments, the terminal further displays any zoomed-in pathological sub-image on the basis of any of the above embodiments, and the detailed processes are referred to the following embodiments of FIG. 17. FIG. 17 is a flowchart of another method for displaying images according to some embodiments of the present disclosure. An execution body of the embodiments of the present disclosure is the terminal, and the embodiments of the present disclosure are described by taking the target type including a plurality of sub-types as an example. Referring to FIG. 17, the method includes the following processes.

In S1701, the terminal displays an object information interface, wherein the object information interface includes at least one object.

In S1702, the terminal acquires a pathological image of a target object in response to a select operation on the target object on the object information interface.

In S1703, the terminal determines at least one pathological sub-image of a target type from the pathological image.

The processes of S1701 and S1703 are the same as the processes of S501 to S503, which is not repeated herein.

In S 1704, the terminal displays the at least one pathological sub-image of the target type on the object information interface.

In some embodiments, the terminal displays the at least one pathological sub-image of the target type on the object information interface, and displays a confidence level indicator of the at least one pathological sub-image. The confidence level indicator of the at least one pathological sub-image indicates a confidence level of the at least one pathological sub-image, and the confidence level of the at least one pathological sub-image indicates a credibility that the pathological sub-image is of the target type.

In some embodiments, the confidence level indicator is a color of the frame of the pathological sub-image. For example, the terminal classifies the confidence levels as a plurality of levels according to the confidence levels of the pathological sub-images, and different levels of the confidence levels correspond to different colors. For example, the confidence levels less than a third threshold are determined as a low level, the confidence levels not less than the third threshold and less than a fourth threshold are determined as a middle level, and the confidence levels not less than the fourth threshold are determined as a high level. The frames of the pathological sub-images with the low level of the confidence levels are green, the frames of the pathological sub-images with the middle level of the confidence levels are yellow, and the frames of the pathological sub-images with the high level of the confidence levels are red.

In addition, the process of the manner of displaying the pathological sub-images in S1704 is the same as the process of the manner of displaying the pathological sub-images in S504, S1204 to S1204, or S1504, which is not repeated herein.

In S1705, the terminal displays a first pathological sub-image zoomed-in by a target magnification in response to a select operation on the displayed first pathological sub-image.

The first pathological sub-image is any of the currently displayed pathological sub-images. The select operation on the first pathological sub-image is configured to request the display of the zoomed-in first pathological sub-image. Thus, in the case that the user wants to view the zoomed-in first pathological sub-image, the user performs the select operation on the first pathological sub-image. The terminal displays the first pathological sub-image zoomed-in by the target magnification in response to a select operation on the first pathological sub-image. In some embodiments, the target magnification is a predetermined magnification, for example, 2, 2.5, and the like.

In some embodiments, the terminal displays the first pathological sub-image zoomed-in by the target magnification in response to the select operation on the displayed first pathological sub-image in the following three zooming-in manners.

In a first zooming-in manner, the terminal displays the first pathological sub-image zoomed-in by the target magnification in response to a touch operation on the displayed first pathological sub-image.

In some embodiments, the touch operation is a tap operation, a drag operation, and the like, which is not limited in the embodiments of the present disclosure.

In a second zooming-in manner, the object information interface further includes an image identifier of the at least one pathological sub-image, and the terminal displays the first pathological sub-image zoomed-in by the target magnification in response to the touch operation on the image identifier of the at least one pathological sub-image.

In a third zooming-in manner, the object information interface further includes an zooming-in option for the at least one pathological sub-image, and the terminal displays the first pathological sub-image zoomed-in by the target magnification in response to the touch operation on the zooming-in option for the at least one pathological sub-image.

For example, as shown in FIG. 16, a left side in the FIG. 16 is the pathological image, regions of the frames in the pathological images are regions of the pathological sub-images, a right side is the image identifiers of the pathological sub-images and the corresponding zooming-in option of "view." The image identifier includes the target type of the pathological sub-image and the corresponding confidence level, the image identifier corresponds to the pathological sub-image, and the zooming-in option also corresponds to the pathological sub-image. In the case that the user performs the tap operation on the image identifier, the terminal determines the pathological sub-image corresponding to the image identifier, and displays the pathological sub-image zoomed-in by the target magnification. In some embodiments, in the case that the user performs the tap operation on the zooming-in option, the terminal determines the pathological sub-image corresponding to the zooming-in option, and displays the pathological sub-image zoomed-in by the target magnification.

In some embodiments, the terminal displays image information of the first pathological sub-image in a first region in the zoomed-in first pathological sub-image. The image information includes at least one of the target type of the first pathological sub-image and a confidence level of the first pathological sub-image. In some embodiments, the first region is a center region in the first pathological sub-image, a region close to an upper edge in the first pathological sub-image, a region close to a left edge in the first pathological sub-image, and the like.

In some embodiments, the terminal displays, in response to a display duration of the image information in the first region being equal to a target duration, the image information in a second region outside the zoomed-in first pathological sub-image. A size of the second region is less than a size of the first region. In some embodiments, the target duration is a predetermined duration, for example, three seconds.

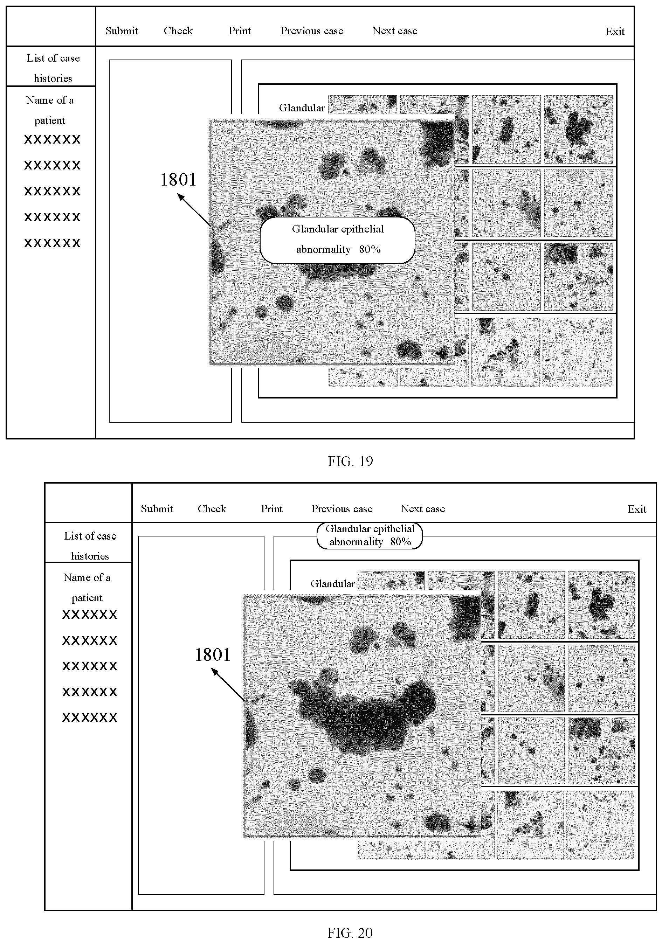

FIG. 18 is a schematic diagram of another object information interface according to some embodiments of the present disclosure. As shown in FIG. 18, a plurality of pathological sub-images are displayed on the object information interface, and the terminal displays the object information interface shown in FIG. 19 in response to the tap operation on the pathological sub-image 1801 by the user. As shown in FIG. 19, the zoomed-in pathological sub-image 1801 is displayed on the object information interface, and the image information "80% glandular epithelial abnormality" corresponding to the pathological sub-image 1801 is displayed in the first region in the pathological sub-image 1801. In the case that the display duration of the image information in the first region is equal to three seconds, the terminal displays the object information interface shown in FIG. 20. As shown in FIG. 20, the zoomed-in pathological sub-image 1801 is displayed on the object information interface, the image information "80% glandular epithelial abnormality" corresponding to the pathological sub-image 1801 is displayed in the second region outside the pathological sub-image 1801, and the image information "80% glandular epithelial abnormality" is displayed in a top region of the object information interface in response to the zooming-out of the image information from the pathological sub-image 1801.

In the method in the embodiments of the present disclosure, at least one object is displayed on the object information interface, and the pathological image of the target object is acquired in response to the select operation on the target object. At least one pathological sub-image of a target type is determined from the pathological image and is displayed, such that key information with higher reference value in the pathological image is displayed, the pertinence of displaying the pathological image is improved, and the display effect of the pathological image is greater.

In addition, the pathological sub-image zoomed-in by the target magnification is displayed in response to the select operation on the displayed pathological sub-image, such that the flexibility of display of the pathological sub-images is improved.

In addition, the image information of the first pathological sub-image is further displayed in the first region in the zoomed-in first pathological sub-image, such that the amount of the displayed information is increased.

Pathological laboratory medicine is becoming the "five modernizations", that is standardization, automation, informatization, humanization, and clinical application. The high-definition and high-resolution pathological images achieve the conversion from optical microscopic images to digital microscopic images, and the pathological images and the pathological image analysis technology are gradually applied.

In some practices, the physician views the pathological images with the naked eye and analyses and diagnoses based on experience, different physicians determine different results for the same pathological image, and it is difficult to view the pathological phenomenon in the pathological image roundly as the limitation of the difference between the naked eyes. In addition, the qualities of the pathological images are different, the pathological image includes disordered and useless regions, or the scanned pathological image includes unclear regions. Thus, the physician fails to quickly and accurately determine key information in the pathological image.

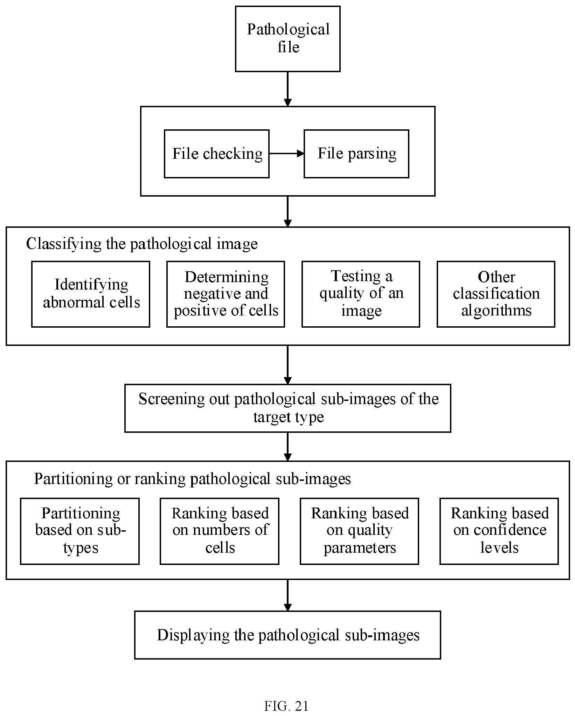

In the method for displaying images in the embodiments of the present disclosure, the key information in the pathological image is displayed to the user. FIG. 21 is a flowchart of another method for displaying images according to some embodiments of the present disclosure. As shown in FIG. 21, the method includes the following five stages.
(1) A file checking and parsing stage: the terminal checks the pathological file upon acquiring the pathological file, and parses the pathological file in the case that the pathological file is successfully checked, such that a pathological image is acquired.
(2) Classifying the pathological image: the terminal selects regions of the target type from the pathological image by various pathological image classification algorithms, for example, the abnormal cell identification algorithm, the cell negative and positive determination algorithm, the image quality test algorithm, and the like.
(3) Screening out pathological sub-images of the target type: the terminal screens out the regions of the target type from the pathological image to acquire the pathological sub-images of the target type.
(4) Partitioning or ranking pathological sub-images: the terminal partitions based on the sub-types of the pathological sub-images, or ranks based on the quality parameters, the numbers of the cells, or the confidence levels.
(5) Displaying the pathological sub-images: the terminal displays the partitioned or ranked pathological sub-images.

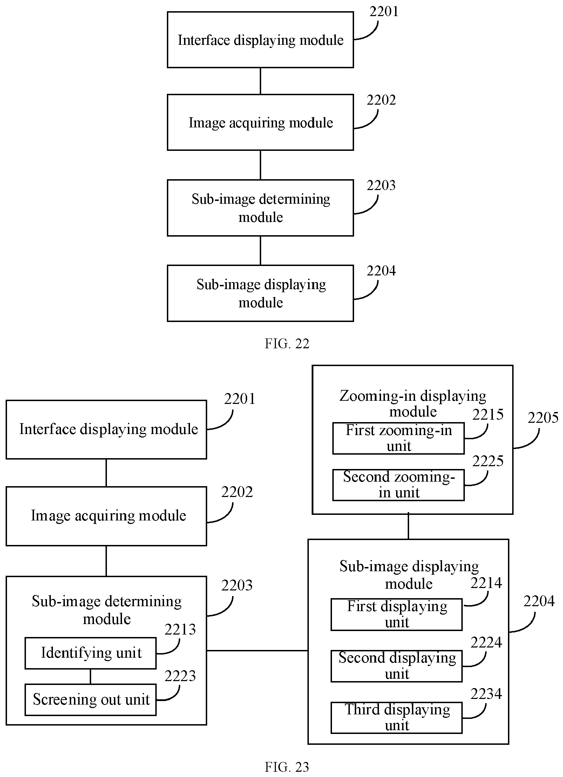

FIG. 22 is a schematic structural diagram of an apparatus for displaying images according to some embodiments of the present disclosure. Referring to FIG. 22, the apparatus includes:
an interface displaying module 2201, configured to display an object information interface, wherein the object information interface includes at least one object;
an image acquiring module 2202, configured to acquire a pathological image of a target object in response to a select operation on the target object on the object information interface;
a sub-image determining module 2203, configured to determine at least one pathological sub-image of a target type from the pathological image; and
a sub-image displaying module 2204, configured to display the at least one pathological sub-image of the target type on the object information interface.

In the apparatus in the embodiments of the present disclosure, at least one object is displayed on the object information interface, and the pathological image of the target object is acquired in response to the select operation on the target object. At least one pathological sub-image of a target type is determined from the pathological image and is displayed, such that key information with higher reference value in the pathological image is displayed, pertinence of displaying the pathological image is improved, and the display effect of the pathological image is greater.

In some embodiments, referring to FIG. 23, the sub-image displaying module 2204 includes:
a first displaying unit 2214, configured to display the at least one pathological sub-image of the target type in an image display region of the object information interface; or
a second displaying unit 2224, configured to display the pathological image on the object information interface, and display a frame of the at least one pathological sub-image in the pathological image.

In some embodiments, referring to FIG. 23, the first displaying unit 2214 is configured to:
in the case that the target type includes a plurality of sub-types, display a plurality of pathological sub-images in partitions according to different sub-types of the plurality of pathological sub-images;
display a plurality of pathological sub-images in an order of numbers of cells in the plurality of pathological sub-images;
display a plurality of pathological sub-images in an order of quality parameters of the plurality of pathological sub-images; or
display a plurality of pathological sub-images in an order of confidence levels of the plurality of pathological sub-images, wherein the confidence level of the pathological sub-image indicates a credibility that the pathological sub-image is of the target type.

In some embodiments, referring to FIG. 23, the target type includes a plurality of sub-types, a number of pathological sub-images of any of the plurality of sub-types in the image display region is not greater than a target number, and the first displaying unit 2214 is configured to:
cancel displaying of pathological sub-images in sub-types other than a first sub-type of the plurality of sub-types in the image display region in response to an expand operation on the first sub-type, and display each of pathological sub-images of the first sub-type.

In some embodiments, referring to FIG. 23, the apparatus further includes:
an zooming-in displaying module 2205, configured to display a first pathological sub-image zoomed-in by a target magnification in response to a select operation on the displayed first pathological sub-image.

In some embodiments, referring to FIG. 23, the zooming-in displaying module 2205 is further configured to:
display image information of the first pathological sub-image in a first region in the zoomed-in first pathological sub-image, wherein the image information includes at least one of the target type of the first pathological sub-image and a confidence level of the first pathological sub-image.

In some embodiments, referring to FIG. 23, the zooming-in displaying module 2205 is further configured to:
display, in response to a display duration of the image information in the first region being equal to a target duration, the image information in a second region outside the zoomed-in first pathological sub-image,
wherein a size of the second region is less than a size of the first region.

In some embodiments, referring to FIG. 23, the zooming-in displaying module 2205 further includes:
a first zooming-in unit 2215, configured to display the first pathological sub-image zoomed-in by the target magnification in response to a touch operation on the first pathological sub-image; or
a second zooming-in unit 2225, configured to: in the case that the object information interface further includes an image identifier of the at least one pathological sub-image, display the first pathological sub-image zoomed-in by the target magnification in response to a touch operation on the image identifier of the at least one pathological sub-image.

In some embodiments, referring to FIG. 23, the sub-image displaying module 2204 includes:
a third displaying unit 2234, configured to display the at least one pathological sub-image of the target type on the object information interface, and display a confidence level indicator of the at least one pathological sub-image, wherein the confidence level indicator of the at least one pathological sub-image indicates a confidence level of the at least one pathological sub-image, and the confidence level indicates a credibility that the pathological sub-image is of the target type.

In some embodiments, the sub-image determining module 2203 is configured to at least one of:
determine at least one pathological sub-image including abnormal cells from the pathological image;
determine at least one pathological sub-image including cells in a target state from the pathological image, wherein the target state includes a negative state and a positive state; and
determine at least one pathological sub-image that s quality parameter meets a target quality condition from the pathological image.

In some embodiments, referring to FIG. 23, the sub-image determining module 2203 includes:
an identifying unit 2213, configured to acquire at least one position information by identifying the pathological image, wherein the position information indicates a region of the target type in the pathological image; and
a screening out unit 2223, configured to screen out a region indicated by the at least one position information from the pathological image, and determine the screened-out region as the at least one pathological sub-image.

In some embodiments, the pathological image is a pathological image of liquid-based cytology or a pathological image of histopathology.

It should be noted that in the case that the apparatus for displaying the images in the embodiments of the present disclosure displays images, divisions of the above functional modules are illustrative. In actual applications, the above functions can be achieved by different functional modules according to actual needs. That is, the inner structure of the terminal is divided to different functional modules to achieve all or part of the above functions. In addition, the apparatus for displaying the images in the embodiments of the present disclosure and the method for displaying images are of the same concept, and thus the detailed implementations are referred to the above embodiments, which are not repeated herein.

Embodiments of the present disclosure further provide a terminal. The terminal includes a processor and a memory storing at least one computer program. The at least one computer program, when loaded and executed by the processor, causes the processor to perform the method for displaying images in the above embodiments.

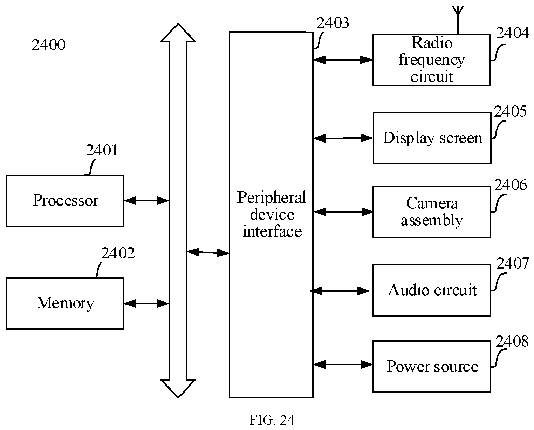

FIG. 24 is a schematic structural diagram of a terminal 2400 according to some embodiments of the present disclosure.

The terminal 2400 includes a processor 2401 and a memory 2402. The processor 2401 includes one or more processing cores, such as a 4-core processor, and an 8-core processor. The processor 2401 is implemented by at least one hardware of the digital signal processor (DSP), the field-programmable gate array (FPGA), or the programmable logic array (PLA). The processor 2401 further includes a primary processor and a coprocessor. The main processor is a processor configured to process the data in an awake state, and is also referred to as a central processing unit (CPU). The coprocessor is a low-power-consumption processor configured to process the data in a standby state. In some embodiments, the processor 2401 is integrated with a graphics processing unit (GPU), which is configured to render and draw the content to be presented by a display screen. In some embodiments, the processor 2401 further includes an artificial intelligence (AI) processor configured to process computational operations related to machine learning.

The memory 2402 includes one or more computer-readable storage mediums. The computer-readable storage medium is non-transitory. The memory 2402 further includes a high-speed random-access memory, and a non-volatile memory, such as one or more disk storage devices and flash storage devices. In some embodiments, a non-transitory computer-readable storage medium in the memory 2402 is configured to store at least one computer program. The at least one computer program, when loaded and executed by the processor 2401, causes the processor 2401 to perform the method for displaying images in the above embodiments.

In some embodiments, the terminal 2400 further includes a peripheral device interface 2403 and at least one peripheral device. The processor 2401, the memory 2402, and the peripheral device interface 2403 are connected by a bus or a signal line. Each peripheral device is connected to the peripheral device interface 2403 by a bus, a signal line, or a circuit board. Specifically, the peripheral device includes at least one of a radio frequency circuit 2404, a display screen 2405, a camera assembly 2406, an audio circuit 2407, and a power source 2408.

The peripheral device interface 2403 is configured to connect at least one peripheral device associated with an input/output (I/O) to the processor 2401 and the memory 2402. In some embodiments, the processor 2401, the memory 2402, and the peripheral device interface 2403 are integrated on the same chip or circuit board. In some embodiments, any one or two of the processors 2401, the memory 2402, and the peripheral device interface 2403 is implemented on a separate chip or circuit board, which is not limited in the embodiments of the present disclosure.

The radio frequency circuit 2404 is configured to receive and transmit a radio frequency (RF) signal, which is also referred to as an electromagnetic signal. The radio frequency circuit 2404 communicates with a communication network and other communication devices via the electromagnetic signal. The radio frequency circuit 2404 converts an electrical signal into the electromagnetic signal to transmit, or converts the received electromagnetic signal into the electrical signal. In some embodiments, the RF circuit 2404 includes an antenna system, an RF transceiver, one or more amplifiers, a tuner, an oscillator, a digital signal processor, a codec chipset, a subscriber identity module card, and the like. The RF circuit 2404 is communicated with other devices via at least one wireless communication protocol. The wireless communication protocol includes, but is not limited to, a metropolitan area network (MAN), various generations of mobile communication networks (2G, 3G, 4G, and 5G), a wireless local area network, and/or a wireless fidelity (Wi-Fi) network. In some embodiments, the RF circuit 2404 further includes near-field communication (NFC) related circuits, which is not limited in the present disclosure.

The display screen 2405 is configured to present a user interface (UI). The UI includes graphics, text, icons, videos, and any combination thereof. In the case that the display screen 2405 is a touch display screen, the display screen 2405 further possesses a capacity of acquiring touch signals on or over the surface of the display screen 2405. The touch signal is input into the processor 2401 as a control signal for processing. In this case, the display screen 2405 is further configured to provide virtual buttons and/or virtual keyboards, which are also referred to as soft buttons and/or soft keyboards. In some embodiments, one display screen 2405 is defined, and is disposed on the front panel of the terminal 2400. In some other embodiments, at least two display screens 2405 are defined, and are disposed on different surfaces of the terminal 2400 or in a folded design. In some embodiments, the display screen 2405 is a flexible display screen disposed on the curved or folded surface of the terminal 2400. Even the display screen 2405 is disposed as an irregular shape other than a rectangle. That is, the display screen 2405 is an irregular-shaped screen. In some embodiments, the display screen 2405 is prepared from a material such as a liquid crystal display (LCD), an organic light-emitting diode (OLED), or the like.

The camera assembly 2406 is configured to capture images or videos. In some embodiments, the camera component 2406 includes a front camera and a rear camera. Generally, the front camera is disposed on the front panel of the terminal, and the rear camera is disposed on the back of the terminal. In some embodiments, at least two the rear cameras are defined, and are any one of a primary camera, a depth-of-field camera, a wide-angle camera, and a telephoto camera, such that a background blurring function is achieved by fusion of the main camera and the depth-of-field camera, panoramic shooting and virtual reality (VR) shooting functions or other fusion shooting functions are achieved by fusion of the main camera and the wide-angle camera. In some embodiments, the camera component 2406 further includes a flashlight. The flashlight is a mono-color temperature flashlight or a two-color temperature flashlight. The two-color temperature flash is a combination of a warm flashlight and a cold flashlight, and is used for light compensation at different color temperatures.

The audio circuit 2407 includes a microphone and a speaker. The microphone is configured to acquire sound waves of users and environments, and convert the sound waves into electrical signals for inputting into the processor 2401 for processing, or inputting into the RF circuit 2404 for voice communication. For the purpose of stereo acquisition or noise reduction, a plurality of microphones are defined, and are disposed at different locations of the terminal 2400. The microphone is also an array microphone or an omnidirectional acquisition microphone. The speaker is configured to convert the electrical signals from the processor 2401 or the RF circuit 2404 into the sound waves. The speaker is a conventional thin film speaker or a piezoelectric ceramic speaker. In the case that the speaker is the piezoelectric ceramic speaker, the electrical signal is converted into human-audible sound waves and the sound waves which are inaudible to humans for ranging and the like. In some embodiments, the audio circuit 2407 further includes a headphone jack.

The power source 2408 is configured to power up various components in the terminal 2400. The power source 2408 is an alternating current, a direct current, a disposable battery, or a rechargeable battery. In the case that the power source 2408 includes the rechargeable battery, the rechargeable battery supports wired charging or wireless charging. The rechargeable battery further supports the fast-charging technology.

It should be understood those skilled in the art that the structure shown in FIG. 24 does not limit the terminal 2400, and the terminal may include more or less assemblies than those shown in the drawing, or combine some assemblies, or disposed in different assemblies.

The embodiments of the present disclosure further provide a computer-readable storage medium. The computer-readable storage medium stores at least one computer program, wherein the at least one computer program, when loaded and run by a processor, causes the processor to perform the method for displaying images in the above embodiments.

The embodiments of the present disclosure further provide a computer program product. The computer program product includes computer programs, wherein the computer programs, when loaded and run by a processor, causes the processor to perform the method for displaying images in the above embodiments. In some embodiments, the computer programs in the embodiments of the present disclosure are run on a computer device, a plurality of computer devices at a same location, or a plurality of computer devices at different locations and communicated with each other over the network. The plurality of computer devices at different locations and communicated with each other over the network form a block chain system.

It should be understood by those of ordinary skill in the art that all or part processes of the above embodiments are performed by the hardware or by instructing the related hardware by the programs. The programs are stored in the computer-readable storage medium that are the read-only memory, the disk, the compact disc, and the like.

Described above are merely exemplary embodiments of the present disclosure, and are not intended to limit the present disclosure. Any modifications, equivalent replacements, improvements and the like made within the spirit and principles of the present disclosure should be encompassed within the scope of protection of the present disclosure.

## Claims

1. A method for displaying images, comprising:
displaying an object information interface, wherein the object information interface comprises at least one object;
acquiring a pathological image of a target object in response to a select operation on the target object on the object information interface;
determining at least one pathological sub-image of a target type from the pathological image; and
displaying the at least one pathological sub-image of the target type on the object information interface.

2. The method according to claim 1, wherein displaying the at least one pathological sub-image of the target type on the object information interface comprises:
displaying the at least one pathological sub-image of the target type in an image display region of the object information interface; or
displaying the pathological image on the object information interface, and displaying a frame of the at least one pathological sub-image in the pathological image.

3. The method according to claim 2, wherein displaying the at least one pathological sub-image of the target type in the image display region of the object information interface comprises:
the target type comprising a plurality of sub-types, and displaying a plurality of pathological sub-images in partitions according to different sub-types of the plurality of pathological sub-images;
displaying a plurality of pathological sub-images in an order of numbers of cells in the plurality of pathological sub-images;
displaying a plurality of pathological sub-images in an order of quality parameters of the plurality of pathological sub-images; or
displaying a plurality of pathological sub-images in an order of confidence levels of the plurality of pathological sub-images, wherein the confidence level of the pathological sub-image indicates a credibility that the pathological sub-image is of the target type.

4. The method according to claim 2, wherein
the target type comprises a plurality of sub-types, and a number of pathological sub-images of any of the plurality of sub-types in the image display region is not greater than a target number; and
the method further comprises:
cancelling displaying of pathological sub-images of sub-types other than a first sub-type of the plurality of sub-types in the image display region in response to an expand operation on the first sub-type, and displaying each of pathological sub-images of the first sub-type.

5. The method according to claim 1, wherein upon displaying the at least one pathological sub-image of the target type on the object information interface, the method further comprises:
displaying a first pathological sub-image zoomed-in by a target magnification in response to a select operation on the displayed first pathological sub-image.

6. The method according to claim 5, further comprising:
displaying image information of the first pathological sub-image in a first region in the zoomed-in first pathological sub-image, wherein the image information comprises at least one of the target type of the first pathological sub-image and a confidence level of the first pathological sub-image.

7. The method according to claim 6, further comprising:
displaying, in response to a display duration of the image information in the first region being equal to a target duration, the image information in a second region outside the zoomed-in first pathological sub-image, wherein a size of the second region is less than a size of the first region.

8. The method according to claim 5, wherein displaying the first pathological sub-image zoomed-in by the target magnification in response to the select operation on the displayed first pathological sub-image comprises:
displaying the first pathological sub-image zoomed-in by the target magnification in response to a touch operation on the first pathological sub-image; or
the object information interface further comprising an image identifier of the at least one pathological sub-image, and displaying the first pathological sub-image zoomed-in by the target magnification in response to a touch operation on the image identifier of the at least one pathological sub-image.

9. The method according to claim 1, wherein displaying the at least one pathological sub-image of the target type on the object information interface comprises:
displaying the at least one pathological sub-image of the target type on the object information interface, and displaying a confidence level indicator of the at least one pathological sub-image, wherein the confidence level indicator of the at least one pathological sub-image indicates a confidence level of the at least one pathological sub-image, and the confidence level indicates a credibility that the pathological sub-image is of the target type.

10. The method according to any one of claims 1 to 9, wherein determining the at least one pathological sub-image of the target type from the pathological image comprises at least one of:
determining at least one pathological sub-image comprising abnormal cells from the pathological image;
determining at least one pathological sub-image comprising cells in a target state from the pathological image, wherein the target state comprises a negative state and a positive state; and
determining at least one pathological sub-image that a quality parameter meets a target quality condition from the pathological image.

11. The method according to any one of claims 1 to 9, wherein determining the at least one pathological sub-image of the target type from the pathological image comprises:
acquiring at least one position information by identifying the pathological image, wherein the position information indicates a region of the target type in the pathological image; and
screening out a region indicated by the at least one position information from the pathological image, and determining the screened-out region as the at least one pathological sub-image.

12. The method according to any one of claims 1 to 9, wherein the pathological image is a pathological image of liquid-based cytology or a pathological image of histopathology.

13. An apparatus for displaying images, comprising:
an interface displaying module, configured to display an object information interface, wherein the object information interface comprises at least one object;
an image acquiring module, configured to acquire a pathological image of a target object in response to a select operation on the target object on the object information interface;
a sub-image determining module, configured to determine at least one pathological sub-image of a target type from the pathological image; and
a sub-image displaying module, configured to display the at least one pathological sub-image of the target type on the object information interface.

14. A terminal, comprising a processor and a memory storing at least one computer program, wherein the processor, when loading and running the at least one computer program, is caused to perform the method for displaying images as defined in any one of claims 1 to 12.

15. A computer-readable storage medium, storing at least one computer program, wherein the at least one computer program, when loaded and run by a processor, causes the processor to perform the method for displaying images as defined in any one of claims 1 to 12.

16. A computer program product, comprising at least one computer program, wherein the at least one computer program, when loaded and run by a processor, causes the processor to perform the method for displaying images as defined in any one of claims 1 to 12.
